# EUROPEAN PATENT APPLICATION

(11) **EP 1 757 296 A1**
(43) Date of publication of application: **28.02.2007**
(21) Application number: 05743550.5
(22) Date of filing: 26.05.2005
(51) Int. Cl.: A61K 31/7004, A23K 1/16, A23L 1/30, A61P 19/02, A61P 27/02, A61P 35/00, A61P 43/00, C07H 3/02, C07H 3/08

(54) **METHOD OF CONTROLLING THE PROLIFERATION OF VASCULAR ENDOTHELIAL CELLS AND INHIBITING LUMEN FORMATION**

(30) Priority: 26.05.2004 JP 2004155659
(71) Applicant: National University Corporation Kagawa University, Takamatsu, Kagawa 760-8521 (JP)
(72) Inventor: TOKUDA, M., Faculty of Medicine, Kagawa University, Kitagun, Kagawa 7610793 (JP); TSUKAMOTO, I., Faculty of Medicine, Kagawa Univ., Kitagun, Kagawa7610793 (JP); KONISHI, R., Faculty of Medicine, Kagawa Univ., Kitagun, Kagawa 7610793 (JP); KUBOTA, Y., Faculty of Medicine, Kagawa University, Kitagun, Kagawa 7610793 (JP); IZUMORI, Ken, Rare Sugar Research Center, Kitagun, Kagawa 7610795 (JP)
(74) Representative: TBK-Patent
(86) International application number: PCT/JP2005/009689
(87) International publication number: WO 2005/115408

(57) **Abstract**

[PROBLEMS] To find out a specific rare sugar having effects of inhibiting the proliferation of vascular endothelial cells and lumen formation and utilize these effects. To provide this rare sugar as a preventive/remedy for diseases with angiogenesis, a cosmetic or a functional food. [MEANS FOR SOLVING PROBLEMS] A method of controlling the proliferation of vascular endothelial cells **characterized by** utilizing the vascular endothelial cell proliferation-controlling effect of D-mannose, D-allose, 2-deoxy-D-glucose, 3-deoxy-D-glucose, L-sorbose, 2-deoxy-D-ribose and/or 2-deoxy-L-ribose. A method of inhibiting lumen formation of vascular endothelial cells **characterized by** utilizing the vascular endothelial cell lumen formation-inhibiting effect of D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose.

## Description

### Technical Field

The present invention relates to a technology concerning rare sugars which have a function suppressing the growth of vascular endothelial cells and/or inhibiting luminal formation and monosaccharides which have the same function as the rare sugars.

### Background Art

Vasculogenesis is a phenomenon in which a new blood vessel is generated, and it relates not only to formation of circulatory organs and histogenesis during the embryonal period of vertebrates but also to luteinization and placentation in the estrus cycle of adult individuals. In addition to these vasculogeneses, on the other hand, pathological vasculogensis which is deeply involved in development of the disease is recognized in chronic inflammation, diabetic retinopathy, or a serious disease taking eyesight such as age-related macular degeneration, or in growth of solid tumor, pannus formation in chronic rheumatoid arthritis, particularly in cavitas articularis, or at the time of growth of the synovial membrane in arthrosis. Thus, the inhibition of such a pathological vasculogenesis is likely to result in cure of those diseases.

The cells covering the lumen in the blood vessel are designated as vascular endothelia cells, which on irritation with a growth factor, physiologically active substance or physical injury are differentiated and proliferated to cause vasculogenesis. A number of growth factors such as vascular endothelial growth factor (VEGF) and fibroblast growth factor (FGF) have been known as the growth factors to directly or indirectly stimulate the growth of vascular endothelial cells. The course of luminal formation with the endothelial cells is one of the courses of vasculogenesis, and a known substance inhibiting vasculogenesis includes peptides or proteins such as cytokines, and secondary metabolites by microorganisms, which are suggested to potentially inhibit the luminal formation of endothelial cells (non-patent document 1). As vasculogenesis inhibitors, a large number of compounds such as fumagillin and its derivative TNP-470, anti-VEGF antibody, a certain VEGF receptor kinase inhibitor, endostatin, and the like have been researched and developed, and their clinical tests are under progress in human (non-patent document 2). Among them, fumagillin and its derivative TNP-470 are deemed strong in the inhibitory action for the growth of vascular endothelial cells, but their specificity to the vascular endothelial cells is still insufficient for providing a satisfactory vasculogenesis inhibitor.
Non-Patent Document 1: Hiroshi Terano, Saibo Kogaku (Cell Technology), 14, p.426-431 (1995)
Non-Patent Document 2: Cells, 32: 108-112, 2000

### Disclosure of Invention

### Problem to be Solved by the Invention

Vasculogenesis is one of physiological phenomena observed during development of tissues, cure of wounds, or growth or metastasis of cancers, and comprises complicated steps in which new blood vessels are constructed from the existing vessels. Though the whole picture has not yet been clear, it is believed that the vascular endothelial cell serves an important role. On the other hand, D-glucose, one of hexoses, is the most important energy source in organisms and is deeply involved in energy metabolism as well as various physiological effects. Though 24 species of hexoses are known to have the same molecular formula C6H12O6, there are few reports on the comparison of their physiological effects. These D-glucose isomers, since they have similar chemical structure to D-glucose, are expected to have the same effect as D-glucose in organisms or to strengthen or inhibit the effect. Relating to the inhibition of pathological vasculogenesis, they are also expected to be utilized as approved drugs/quasi drugs, cosmetics or functional foods.

In such circumstances, the present inventors have investigated on compounds suppressing the growth of vascular endothelial cell, compounds inhibiting the luminal formation of endothelial cell, particularly whether a rare sugar has such an activity. The purpose of the invention is to find a particular rare sugar exhibiting an inhibitory action for the growth of vascular endothelial cell and the luminal formation and a monosaccharide having the same function as the rare sugar, and utilize thus found action, and to provide the rare sugar as a drug for prevention or treatment of diseases accompanied by vasculogenesis or as cosmetics or functioning foods.

### Means for Carrying Out the Invention

In this situation, the present inventors have investigated the effect of monosaccharides having an influence on the growth of vascular endothelial cell and the luminal formation. Using 20 species of hexoses, 6 species of hexitols and 7 species ofpentoses, their action was compared to elucidate the existence of correlation in the structural activities to find a potential of innovative drug development. The present inventors investigated the effect of monosaccharides exerting an influence on the growth of vascular endothelial cell and the luminal formation, and as a result they found in certain rare sugars a compound showing the action suppressing the growth of vascular endothelial cell and the luminal formation as well as a monosaccharide having the same action as said compound. Thus, the inventors have completed the invention as a drug for prevention or treatment of diseases accompanied by vasculogenesis or as cosmetics or functioning foods.

Briefly, the invention provides the following methods (1) to (4) for suppressing the growth of vascular endothelial cell.
(1) A method for suppressing the growth of vascular endothelial cell which comprises utilizing an inhibitory effect of D-mannose, D-allose, 2-deoxy-D-glucose, 3-deoxy-D-glucose, L-sorbose, 2-deoxy-D-ribose and/or 2-deoxy-L-ribose for the growth of vascular endothelial cell.
(2) A method for suppressing the growth of vascular endothelial cell as described in the above item (1), which comprises utilizing the effect of D-mannose, D-allose, 2-deoxy-D-glucose, 3-deoxy-D-glucose, L-sorbose, 2-deoxy-D-ribose and/or 2-deoxy-L-ribose and/or a derivative thereof and/or a mixture thereof in the form of composition thereof.
(3) A method for suppressing the growth of vascular endothelial cell as described in the above item (2), wherein the above-mentioned composition is in the form selected from the group consisting of sweeteners, flavors, food additives, food materials, foods and beverages, foods and beverages for health, approved drugs/quasi drugs and feeds containing as active ingredient D-mannose, D-allose, 2-deoxy-D-glucose, 3-deoxy-D-glucose, L-sorbose, 2-deoxy-D-ribose and/or 2-deoxy-L-ribose and/or a derivative thereof and/or a mixture thereof.
(4) A method for suppressing the growth of vascular endothelial cell as described in the above item (3), wherein the above-mentioned foods and beverages for health are those used for a patient of a disease accompanied by pathological vasculogenesis.

Briefly, the invention provides the following compositions (5) to (7) having an inhibitory effect for the growth of vascular endothelial cell.
(5) A composition having a suppressive effect for the growth of vascular endothelial cell, which comprises as active ingredient D-mannose, D-allose, 2-deoxy-D-glucose, 3-deoxy-D-glucose, L-sorbose, 2-deoxy-D-ribose and/or 2-deoxy-L-ribose and/or a derivative thereof and/or a mixture thereof.
(6) A composition as described in the above item (5), wherein the above-mentioned composition is in the form selected from the group consisting of sweeteners, flavors, food additives, food materials, foods and beverages, foods and beverages for health, approved drugs/quasi drugs and feeds.
(7) A composition as described in the above item (6),
wherein the above-mentioned foods and beverages for health are those used for a patient of a disease accompanied by pathological vasculogenesis.

Briefly, the invention provides the following methods (8) to (11) for inhibiting the luminal formation of endothelial cell.
(8) A method for inhibiting the luminal formation of endothelial cell which comprises utilizing an inhibitory effect of D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose for the luminal formation of vascular endothelial cell.
(9) A method for inhibiting the luminal formation of endothelial cell as described in the above item (8), which comprisesutilizing the effect of D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose and/or a derivative thereof and/or a mixture thereof in the form of composition thereof.
(10) A method for inhibiting the luminal formation of endothelial cell as described in the above item (9), wherein the above-mentioned composition is in the form selected from the group consisting of sweeteners, flavors, food additives, food materials, foods and beverages, foods and beverages for health, approved drugs/quasi drugs and feeds containing as active ingredient D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose and/or a derivative thereof and/or a mixture thereof.
(11) A method for inhibiting the luminal formation of endothelial cell as described in the above item (10), wherein the above-mentioned foods and beverages for health are those used for a patient of a disease accompanied by pathological vasculogenesis.

Briefly, the invention provides the following compositions (12) to (14) having an inhibitory effect for the luminal formation of vascular endothelial cell.
(12) A composition having an inhibitory effect for the luminal formation of vascular endothelial cell, which comprises as active ingredient D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose and/or a derivative thereof and/or a mixture thereof.
(13) A composition as described in the above item (12), wherein the above-mentioned composition is in the form selected from the group consisting of sweeteners, flavors, food additives, food materials, foods and beverages, foods and beverages for health, approved drugs/quasi drugs and feeds.
(14) A composition as described in the above item (13), wherein the above-mentioned foods and beverages for health are those used for a patient of a disease accompanied by pathological vasculogenesis.

Briefly, the invention provides the following methods (15) to (18) for suppressing an abnormal vasculogenesis.
(15) A method for suppressing an abnormal vasculogenesis which comprises utilizing a suppressive effect of D-mannose, D-allose, 2-deoxy-D-glucose, 3-deoxy-D-glucose, L-sorbose, 2-deoxy-D-ribose and/or 2-deoxy-L-ribose for the growth of vascular endothelial cell, and/or an inhibitory effect of D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose for the luminal formation of vascular endothelial cell.
(16) A method for suppressing an abnormal vasculogenesis as described in the above item (15), which comprises utilizing the effect of D-mannose, D-allose, 2-deoxy-D-glucose, 3-deoxy-D-glucose, L-sorbose, 2-deoxy-D-ribose and/or 2-deoxy-L-ribose and/or a derivative thereof and/or a mixture thereof in the form of composition thereof, and/or the effect of D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose and/or a derivative thereof and/or a mixture thereof in the form of composition thereof.
(17) A method for suppressing an abnormal vasculogenesis as described in the above item (16), wherein the above-mentioned composition is in the form selected from the group consisting of sweeteners, flavors, food additives, food materials, foods and beverages, foods and beverages for health, approved drugs/quasi drugs and feeds containing as active ingredient D-mannose, D-allose, 2-deoxy-D-glucose, 3-deoxy-D-glucose, L-sorbose, 2-deoxy-D-ribose and/or 2-deoxy-L-ribose and/or a derivative thereof and/or a mixture thereof, and/or D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose and/or a derivative thereof and/or a mixture thereof.
(18) A method for suppressing an abnormal vasculogenesis as described in the above item (17) , wherein the above-mentioned foods and beverages for health are those used for a patient of diabetes mellitus.

Briefly, the invention provides the following compositions (19) to (21) for suppressing an abnormal vasculogenesis.
(19) A composition for suppressing an abnormal vasculogenesis, which comprises as active ingredient D-mannose, D-allose, 2-deoxy-D-glucose, 3-deoxy-D-glucose, L-sorbose, 2-deoxy-D-ribose and/or 2-deoxy-L-ribose and/or a derivative thereof and/or a mixture thereof, and/or D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose and/or a derivative thereof and/or a mixture thereof.
(20) A composition as described in the above item (19), wherein the above-mentioned composition is in the form selected from the group consisting of sweeteners, flavors, food additives, food materials, foods and beverages, foods and beverages for health, approved drugs/quasi drugs and feeds.
(21) A composition as described in the above item (20) , wherein the above-mentioned foods and beverages for health are those used for a patient of diabetes mellitus.

### Effect of the Invention

The vaculogenesis inhibitors of the invention exhibited specifically a suppressive effect for the growth of vascular endothelial cell and an inhibitory effect for the luminal formationof vascular endothelial cell. From this observation, these rare sugars and monosaccharides having the same function as the rare sugars are expected to be used as therapeutic agents for treatment of diseases accompanied by pathogenic vasculogenesis such as diabetic retinopathy, or visual decrease orblindness due to age-relatedmacular degeneration, the growth of solid tumor, pannus formation in chronic rheumatoid arthritis, particularly in cavitas articularis, the growth of synovial membrane in arthrosis, or psoriasis.
According to the invention, it is possible to provide rare sugars inhibiting the luminal formation and monosaccharides having the same function as the rare sugars, as well as rare sugars inhibiting the vasculogenesis and monosaccharides having the same function as the rare sugars; these sugars are useful as medicaments and diagnostic agents.
Particularly, the sugars are useful as therapeutic agents for diseases relating vasculogenesis such as cancer, chronic rheumatoid arthritis, diabetic retinopathy, and the like.
Their effect can be utilized not only as approved drugs or quasi drugs but also in the form of sweeteners, flavors, food additives, food materials, foods and beverages, foods and beverages for health, and feeds.

### Best Mode for Carrying Out the Invention

The vascular endothelial cell derived from human used in the inventionmaybe obtained by incubating the cell collected from a human sample in an appropriate medium. The readily available cells which are most frequently used as human-derived vascular endothelial cells are those from the umbilical cord vein, and in the instant invention such cells are conveniently used, though cells derived from other vessel may also be used. Methods for isolation and incubation of the vascular endothelial cell from the umbilical cord vein or from other vessel are well known to a person skilled in the art, and the vascular endothelial cell maybe obtained by any of methods. The cultured cell derived from the endothelial cell of umbilical cord vein is commercially available and can be used conveniently.

In measuring vasculogenesis in the invention, for example, the cord-derived vascular endothelial cell which has been subcultured on a commercially available culture medium containing 2% fetal calf serum for growing endothelial cell may suitably be used, and after recovery the cell subcultured by 2 to 6 passages is preferably used.

As the human-derived fibroblasts in the invention, cells derived from the skin of human adults are preferably used. Methods for isolation and incubation of the fibroblasts from the skin tissues of human adults are well known to a person skilled in the art, and the cell may be obtained by any of methods. In addition, similarly to the vascular endothelial cells, the cultured fibroblasts are commercially available and can be used conveniently.

In measuring vasculogenesis in the invention, for example, the cell which has been subcultured on a commercially available Dulbecco's modified Eagle's medium containing 10% fetal calf serummaysuitablybeused, andparticularlythe cell subcultured by 6 to 10 passages from recovery is preferably used.

The followings describe the rare sugars used in the invention. The "rare sugar" can be defined as a monosaccharide naturally occurring in a trace amount. Monosaccharides abundant in nature include 7 species of sugars such as D-glucose , D-fructose, D-galactose, D-mannose, D-ribose, D-xylose and L-arabinose, and other sugars are classified into rare sugars since they are very few in nature. Naturally occurring sugar alcohols which may be obtained by reduction of monosaccharides include D-sorbitol and D-mannitol which are relatively abundant in nature; other sugar alcohols are not much in quantity and defined as rare sugars according to the invention. Though these rare sugars were difficult to obtain until now, methods for producing such rare sugars from monosaccharides occurring abundantly innature have been developed, in which new technology allows the production of rare sugars.
The following illustration is made based on Izumoring (registered trade mark; hereinafter omitted) proposed for understanding the relationship of these monosaccharides more easily (see: WO 03/097820).

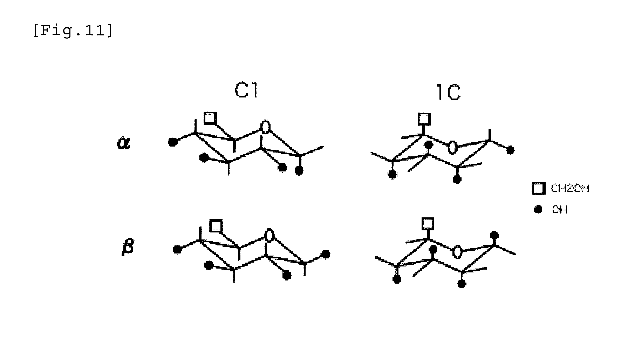

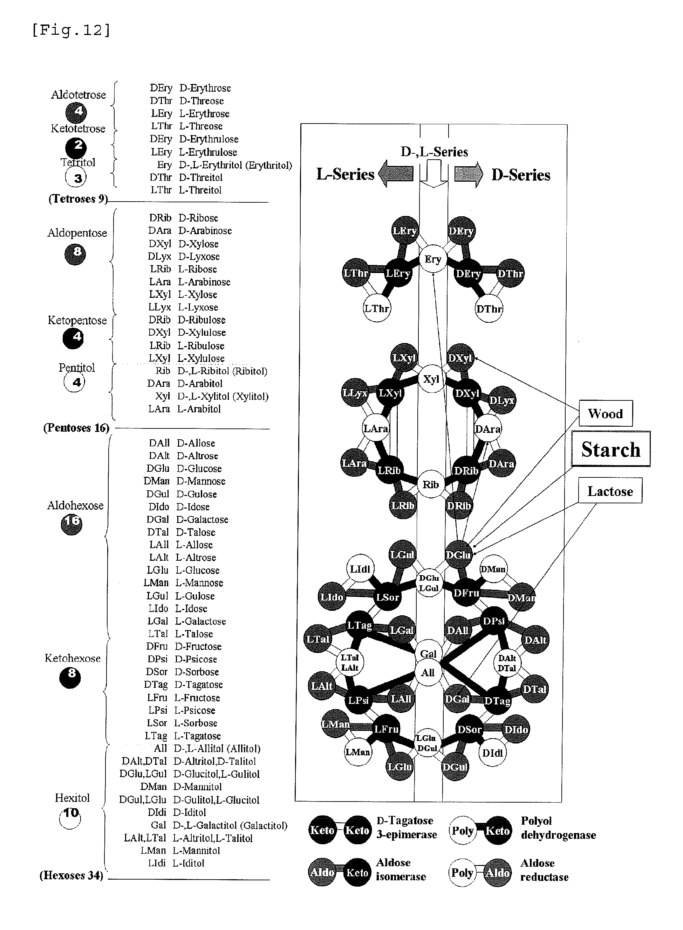

Fig.12 shows an entire view of Izumoring, a coordinated picture, inwhichall of monosaccharides of 4 carbons to 6 carbons are linked together with the course of production and molecular structures (D-form, L-form). That is, it is understood from Fig.12 that the monosaccharides having 4, 5 and 6 carbon atoms are linked all together. The entire view indicates the linkage in Izumoring C6, the linkage in Izumoring C5, the linkage in Izumoring C4, and the linkage in all of C4, C5 and C6. This way of thinking is important. Reduction of the carbon number is achieved by mainly using fermentation. It is also characteristic that all of monosaccharides different in the carbon number are linked in a large coordinated picture.

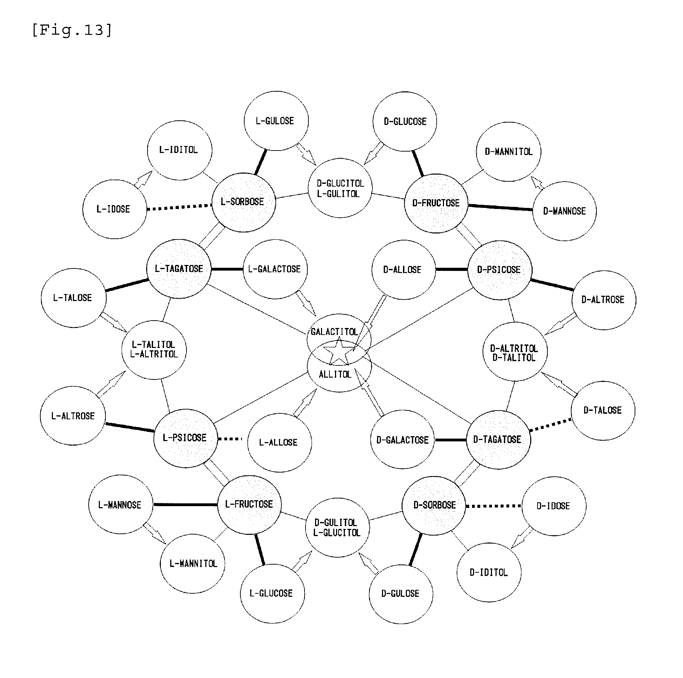

In Izumoring of monosaccharides (hexoses) of 6 carbons, as shown in the lower part of Fig. 12 and in Fig. 13, the monosaccharides (hexoses) of 6 carbons include 34 species of sugars, including 16 species of aldoses, 8 species of ketoses and 10 species of sugar alcohols. It is known that these sugars can be converted by action of an oxidoreductase, action of an aldose isomerase, and action of aldose reductase, as shown even in the study by the present inventors.
In the previous study, however, the upper-side group, the middle group and the lower group were not linked by enzymatic reactions. In other words, though D-glucose (grape sugar) and D-fructose belonging to the upper group exist abundantly in nature and are inexpensive, it was not possible to synthesize rare sugars from them. In the course of the study by the present inventors, however, an enzyme which tied them was found. It arises from the unexpected finding of D-sorbose in the culture medium of a microorganism involving an enzyme which can synthesize D-tagatose from galactitol. As a result of a thorough investigation of the cause, it was found that the microorganism produced an enzyme D-tagatose 3-epimerase (DTE) .
As shown in the lower part of Fig.12 and in Fig.13, it is recognized that the DTE is an enzyme bridging D-tagatose and D-sorbose, which were not bridged previously. More surprisingly, the enzyme DTE can epimerize the 3 position of all of ketoses, and acts on the so far synthetically not connected sugars, i.e., D-fructose and D-psicose, L-sorbose and L-tagatose, D-tagatose and D-sorbose, L-psicose and L-fructose; thus, this enzyme was found to be a unique enzyme having an extremely wide range of substrate specificity. On the basis of the finding of DTE, all of monosaccharides were linked in a cyclic state, leading to the completion of structured knowledge of monosaccharides; thus, this was designated as Izumoring.
Careful examination of Fig.13 indicates that the L-isomers are in the left side, the D-isomers in the right side, and the DL-isomers in the center, and that the L-isomers and the D-isomers are symmetrical at the point of the center (star mark) of the ring. For example, D-glucose and L-glucose are symmetrical at the point of the center. In addition, the value of Izumoring exists in that it is also a plan for production of all of monosaccharides. In the above case, it is indicated that when L-glucose is intended to be produced starting from D-glucose, D-glucose is isomerized, then epimerized, then reduced, then oxidized, then epimerized, and then isomerized to give L-glucose.
By using Izumoring relating to monosaccharides of 6 carbons (hexoses), the relationship between naturally abundant sugars and rare sugars occurring in very small quantity in nature can be illustrated. D-Glucose, D-fructose, D-mannose, and D-galactose produced from lactose in cow milk are abundant in nature, and others existing in very small quantities in nature are classified into rare sugars. Discovery of DTE allowed the production of D-fructose and D-psicose from D-glucose, and further the production of D-allose, allitol and D-tallitol.
In summary, the significance of Izumoring relating to monosaccharides of 6 carbons (hexoses) exists in that all of monosaccharides are structurally rearranged by the production course and the molecular structure (D-isomer and L-isomer) (structured knowledge) to hold a general view of monosaccharides, that an effective and efficient approach for the study can be selected, that an optimal route for production can be designed, and that a deficient part can be foreseen.

D-Glucose in Izumoring C6 is linked to D-arabitol in Izumoring C5 and erythritol in Izumoring C4. This line indicates that D-arabitol and erythritol can be produced from D-glucose by fermentation. That is, Izumoring C6, Izumoring C5 and Izumoring C4 are linked together. This linkage is mainly based on the fermentation reaction inducing reduction of the carbon number; thus, the linkage of Izumoring C6 to Izumoring C5 and C4 is allowed by a fermentation process other than two processes of conversion reaction to D-arabitol and erythritol. For example, D-ribose can be produced from D-glucose. Thus, all of monosaccharides (aldoses, ketoses and sugar alcohols) of 4, 5 and 6 carbons are linked in 3 Izumorings and as a result the existing sites of the respective monosaccharides in all the monosaccharides can be confirmed clearly. In this experiment, the effect to the vascular endothelial cell was tested by using hexoses as well as 5 species of pentoses and 2 species of their deoxy derivatives (total 7 species).

It canclearlybe confirmed that themostprominentxylitol can easily be produced by reducing D-xylose which can be produced from the unused resource woody parts. If a particular monosaccharide is obtained in large quantities by biological reaction, the possibility of conversion of such a raw material into a new monosaccharide could easily be found. That is, the position of all of monosaccharides as raw materials can certainly be confirmed from the general view, and thus a useful method for utilization can be designed. Particularly, when a monosaccharide is obtained from waste materials or by-products, a method for utilization can readily be deduced. Not only in a field of rare sugar production, but also in a research of the physiological activity of rare sugars, its efficacy is exhibited. For example, when a physiological activity is found in a certain rare sugar, its existing site is confirmed in the coordinated picture as shown in Fig. 12. And its physiological activity is compared to that of another rare sugar having a very similar structure or to that of a rare sugar which is structurally relating to an enantimer thereof; thus, its examination would assist to deduce a mechanism of the physiological activity from the molecular structure. From analysis of the physiological function of rare sugars and accumulation of their properties on Izumoring, it is expected that the "structure of monosaccharides", "method for production of monosaccharides" and "physiological function of monosaccharides" could be utilized in extensive understanding of the overall monosaccharides, beyond the simple enumerating understanding so for.
The general picture (Fig.12) of Izumoring is a coordinated picture in which all of monosaccharides of 4 carbons to 6 carbons are linked; thus, it is understood that the monosaccharides of 4, 5 and 6 carbons all are linked. In the general picture, the linkage in Izumoring C6, the linkage in Izumoring C5, the linkage in Izumoring C4, and C4, C5 and C6 all are linked. For example, Izumoring of monosaccharides of 6 carbons (hexoses), as shown in the lower part of Fig.12 and in Fig.13, includes 34 species of monosaccharides of 6 carbons (hexoses) in total, including 16 species of aldoses, 8 species of ketoses and 10 species of sugar alcohols.

Among rare sugars, the followings describe a rare sugar D-psicose which now can be produced in large quantities. Psicose is one of hexoses having a keto-group among monosaccharides. Psicose is known to have optical isomers, D-isomer and L-isomer. Though D-psicose is a known substance and defined as "rare sugar" by the International Association of Rare Sugars since it rarely exists in nature. D-Psicose is D-isomer of psicose classified into ketoses among hexoses (C6H1206). Thus, D-psicose may be obtained in any way including extraction from the natural source, and a chemical or biochemical synthesis. For example, it may relatively readily be prepared by means of using an epimerase (e.g., see JP-A 6-125776 gazette). The resulting D-psicose extract, if required, may be purified by means of deproteinization, decoloration, desalting, etc., and further concentrated to give a syrupy D-psicose product; in addition, it may be further purified easily by fractionation by column chromatography to give highly pure specimen of 99% or more purity. Thus, D-psicose is expected to be utilized as monosaccharide as such and if required as a variety of derivatives thereof.

The followings describe D-allose. D-Allose is a rare sugar of which a variety of physiological activities have been recognized in the course of the study of rare sugars. D-Allose (D-allohexose) is a D-isomer of allose classified into aldose (aldohexose) of hexoses (C6H12O6) having mp.of 178°C. As a process for producing D-allose, there are a process in which D-allonic acid lactone is reduced with sodium amalgam and a process in which allose is synthesized from D-psicose using L-rhamnose isomerase (described in Shakewatt Josein Puiyan et al., Journal of Fermentation and Bioengineering, vol. 85, 539-541 (1993)) . Moreover, recent year, it is described in JP-A 2002-17392 gazette. A process for producing D-allose from D-psicose by action of D-xylose isomerase to a solution containing D-psicose has been invented. According to the process as described in JP-A 2002-17392 gazette, when D-allose is produced, D-allose is obtained as an enzymatic reaction solution containing newly produced D-allose together with D-psicose remaining unchanged.
The species of enzymes used in converting a D-allose-convertible substrate into D-allose by action of an enzyme include, but not limited to, "L-rhaminose isomerase" as a preferred enzyme which is able to convert D-psicose into D-allose . L-Rhaminose isomerase is a publicly known enzyme as described in Journal of Fermentation and Bioengineering, vol.85, 539-541 (1998). It is an enzyme that catalyzes the isomerization of L-rhamnose to L-rhamnurose and the isomerization of L-rhamnurose to L-rhamnose. L-Rhamnose isomerase also acts on isomerization between D-allose and D-psicose and can be used in production of D-allose from D-psicose, accordingly.

The invention relates to sweeteners, flavors, food additives, food materials, foods and beverages, foods and beverages for health, approved drugs/quasi drugs and feeds which can be used in prevention or treatment of diseases accompanied by pathological vasculogenesis, for example, diabetic retinopathy, or visual decrease or blindness due to age-related macular degeneration, the growth of solid tumor, pannus formation in chronic rheumatoid arthritis, particularly in cavitas articularis, the growth of synovial membrane in arthrosis, or psoriasis. The prophylactics or therapeutics may be used alone or together with conventional excipients such as general fillers, stabilizers, preservatives, binders, disintegrators, and the like to form a suitable formulation such as liquid preparations, capsules, granules, pills, powders, tablets, and the like, which may be administered orally or rectally. The dosage for oral administration is preferably 0.3 to 50 g/day as D-psicose for an adult (the other sugars used in the invention may be administered according to this range. Hereinafter, the explanation is made with D-psicose as a representative sugar); this dosage may be increased or decreased properly depending on the age and condition. The above daily dosage as a drug for suppressing blood sugar rise may be administered once a day or at suitable intervals divided into 2 or 3 doses a day, or before or after or during the meal.

The feeds of the invention include those provided for domestic animals, domestic fowls, and other feeding animals including honey bees, silk worms, and fishes, and are characterized in that a composition containing D-psicose and/or a derivative thereof and/or a mixture thereof is blended in foods and beverages in an amount of 0.1-50% by weight as D-psicose for the amount of carbohydrate (the amount of saccharide) . When such a feed is given to domestic animals, domestic fowls, and other feeding animals including honey bees, silk worms, and fishes, a tendency to obesity is moderated. Thus, the feeds of the invention are useful in prevention of obesity or diabetes mellitus in pet animals or in keeping meat animals (for human consumption) having fatless meat.

In a process for preparing sweeteners, flavors, food additives, food materials, foods and beverages, foods and beverages for health, approved drugs/quasi drugs and feeds into which a composition comprising D-psicose and/or a derivative thereof and/or a mixture thereof is blended, the composition may be added to them in an amount of 0.1% by weight or more, preferably 0.5% by weight as D-psicose at any time during the course of production of the final product, wherein publicly known methods such as mixing, kneading, dissolving, fusion, immersion, penetration, scattering, application, coating, splaying, infusion, crystallization, solidification, and the like are suitably selected.
In the composition into which D-psicose and/or a derivative thereof and/or a mixture thereof has been blended, the content of D-psicose may be in the range of 0.1 - 50% by weight, preferably 0.5 - 30% by weight, and more preferably 1 - 10% by weight. When the content of D-psicose in the composition is less than 0.1% by weight, the suppressive effect for a sudden rise in blood sugar is insufficient. The content of D-psicose over 50% by weight in the composition is inappropriate in an economical sense. The above description was made on D-psicose as a typical example, but the same will be applied to other rare sugars and monosaccharides having the same function while keeping their characteristics.

The followings describe in detail the pharmaceutical preparations containing the rare sugars of the invention or monosaccharides having the same function, derivatives or pharmaceutically acceptable salts thereof, or/and hydrates (hereinafter referred to as the compound of the invention).
As for the formulation as vasculogenesis inhibitors of the invention, avarietyof forms containing anactive ingredient together with additives such as medically acceptable carriers, fillers, lubricants and binders, for example, liquid preparations dissolved in water or various transfusions, powders, granules, tablets, injections, suppositories, or external preparations can be prepared in a publicly known pharmaceutical technique. When the compound of the invention is used as a drug for the human organism, the dosage is generally in the range of 0.1 - 100 mg/day in oral administration for an adult, usually 0. 1 - 10 mg/day, and in some cases it is preferred to slowly decrease the dose. For administration by injection, the dosage is usually 1/5 of that for oral administration and may be slowly increased or decreased if required. When administeredlocallyto focus, e.g., joint cavity or ophthalmus, the dosage may further be reduced to reduce the action to the whole body and to use effectively. The dosage is variable depending on the condition, age, or body weight of the patient.

When the rare sugars of the invention or monosaccharides having the same function are administered by injection, aqueous injection, aqueous suspension for injection, fat emulsion, or liposome injection, is preferred. The aqueous injection or aqueous suspension for injection may be prepared by mixing a rare sugar of the invention, a derivative or pharmaceutically acceptable salt thereof with pure water, then if required adding a water-soluble or water-swelling macromolecule, pH regulator, surface activator, osmotic pressure-controller, antiseptic, or preservative, then mixing, then if required dissolving or suspending the mixture under heating, then sterilizing, and filling and sealing into vessels for injection use to give an aqueous injection or aqueous suspension for injection. The aqueous injection may be administered intravenously, subcutaneously, intramuscularly, intracutaneously, or in joint cavity. In addition, the aqueous suspension for injection may be applied subcutaneously, intramuscularly, intracutaneously, or in joint cavity. Oral administration is also allowed.

Water-soluble or water-swelling macromolecule includes gelatin, cellulose derivatives, acrylic acid derivatives, povidone, macrogol, polyamino acid derivative, or polysaccharide, preferably; gelatin is preferably used as purified gelatin; cellulose derivative includes preferably methylcellulose, hydroxypropylmethyl cellulose 2910, hydroxypropylmethyl cellulose 2208, hydroxypropylmethyl cellulose 2906, hydroxypropyl cellulose, lower substituted hydroxypropyl cellulose, carmellose sodium; acrylic acid derivative includes preferably aminoacryl methacrylate copolymer, methacrylic acid copolymer; and polyamino acid derivative preferably includes polylysine, and polyglutamic acid. The particularly preferred polysaccharide includes hyarulonic acid, dextran or dextrin. The amount of water-soluble or water-swelling macromolecule to be added is variable depending on the properties and amount of escretin, derivatives thereof, or pharmacologically acceptable salts thereof, as well as the properties, molecular weight and applied site of water-soluble or water-swelling macromolecule, and in general it may be used in the range of 0.01% to 10% for the total preparation.

As a pH regulator, an acid or alkali innocuous to the human body is used; and as a surface activator, a non-ionic surface activator, anionic surface activator or amphoteric surface activator is used. An osmotic pressure-controller is exemplified by sodium chloride, glucose, etc.; an antiseptic is exemplified by paraben, etc.; and a preservative is exemplified by ascorbic acid and sulfites. The amount of these additives to be used is not particularly limited and may be used in the range in which their action is displayed. In addition, if required, a local anesthetic agent such as procaine hydrochloride, a soothing agent such as benzyl alcohol, chelating agent, a buffering agent, or a water- soluble organic solvent may be added.

Fat emulsion may be prepared by blending an emulsifying agent and a rare sugar, a derivative thereof or a pharmacologically acceptable salt thereof, to suitable fat and oil, then adding purified water thereto, and then if required adding a water-soluble or water-swelling macromolecule, pH regulator, surface activator, osmotic pressure-controller, antiseptic, or preservative, then emulsifying it in a suitable emulsifying apparatus, then sterilizing, and filling and sealing into vessels for injection use.

The oral preparation containing the compound of the invention may be selected according to an administration way from the formulations containing the compound of the invention as a maj or ingredient, for example, tablets, powders, granules, capsules, solutions, syrups, elixirs, or oily or aqueous suspensions; these preparations may be prepared together with additives such as conventional fillers, lubricants and binders, according to the publicly known technology.
The solid preparations contain a pharmaceutically acceptable excipients together with an active compound of the invention, and may be mixed and formulated with, for example, fillers or extenders, binders, disintegrators, dissolution accelerators, wetting agents, or wetting lubricants, if necessary.
The external preparations are exemplified by solutions, suspensions, emulsions, ointments, gels, creams, lotions, and sprays.

In this invention, the typical compounds suppressing the growth of vascular endothelial cell include D-mannose, D-allose, 2-deoxy-D-glucose, 3-deoxy-D-glucose, L-sorbose, 2-deoxy-D-ribose, 2-deoxy-L-ribose; and/or the typical compounds inhibiting the luminal formation of endothelial cell include D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose ; these are aldohexose, ketohexose, hexitol, and aldopentose, and they are highly safe compounds administrable to human, accordingly.

The most important and highest hurdle in the development of approved drugs/quasi drugs or foods is to confirm safety of the rare sugar as a new substance. As the most basic safety tests, mutagenicity, biodegradable test and 3 acute toxicity tests (oral acute toxicity test, skin primary irritation test, ophtalmic primary irritation test) are decided. Since rare sugars are monosaccharides naturally occurring in very small quantities, it is necessary to examine exactly, though they are expected to be safe. We requested of a designated organ to examine the basic safety test on 3 species of rare sugars, i.e., D-psicose, D-allose and allitol. As a result, it was confirmed that there was no problem in any of the rare sugars.

The invention will be explained by Examples in detail. The invention is not limited in any way by these Examples.

### Example 1

D-Glucose, one of hexoses, is the most important energy source, and is involved in a variety of physiological effects in addition to energy metabolism. Though 24 species of hexoses having the same molecular formula C₆H₁₂O₆ are known, however, there are few reports on a comparative examination of their physiological activity. These hexoses, which are isomers of D-glucose and have similar chemical structures to D-glucose, accordingly, are expected to have the same effect as D-glucose in vitro, or to strengthen or suppress the effect.
This time, the effects of monosaccharides influenced on the growth of human umbilical cord vascular endothelial cell (HUVEC) and luminal formation were comparatively examined.

### «Materials»

The species and abbreviation of the sugars examined are as follows.

### 15 Species of aldohexoses

D-glucose (G) 2, D-mannose (MAN) 3, D-allose (A) 1 , D-galactose (GAL)3, D-altrose (ALT)1, D-gulose (GUL)2, D-talose (TAO)4, L-glucose (LGL)4, L-mannose (LMA)2, L-allose (LAL)2, L-galactose (LGA)6, 2-deoxy-D-glucose (2DG)2, 3-deoxy-D-glucose (3DG)2, 6-deoxy-D-galactose (DFU)2, 6-deoxy-L-galactose (LFU)2

### 5 Species of ketohexoses

D-fructose (F)2, D-psicose (P)1, D-tagatose (TAG)2, D-sorbose (DSO)4, L-sorbose (SOR)3

### 6 Species of hexitols

allitol (ALL) 1, D-mannitol (MAL)2, dulcitol (=galactitol: DUL)3, D-tallitol (TAL) 1, D-sorbitol (SOL)2, L-iditol (LIL)7

### 7 Species of aldopentoses

D-ribose (RIB)3, D-arabinose (ARA)1, D-lyxose (LYX)5, D-xylose (XYL)2, L-ribose (LRI)2, 2-deoxy-D-ribose (DRI)4, 2-deoxy-L-ribose (LDR)2

These sugars were obtained from Kagawa University Rare Sugar Research Center 1, SIGMA2, WAKO3, ICN Biochemical 4, Tokyo Kasei 5, Avocado Research Chemicals Ltd. 6, and Toronto Research Chemicals Inc. 7, and dissolved in physiological saline to give 400mM aqueous solution, then properly diluted, and added to culture media at final concentrations of 0.1 - 20mM.
Vascular endothelial growth factor A (VEGF) was purchased from KURABO Co.

### «Experiment of growth»

Human umbilical cord vascular endothelial cells (HUVEC) were purchased as primary cultured cells from KURABO (Kurabo Co., Osaka, Japan) and the cells up to P8 were used in experiments. The cells were inoculated on a 96-well plate at a rate of 3000 cells/well, and allowed to stand in Humedia EG2 (Kurabo) overnight (37°C, 5% CO₂). Next morning, the medium was replaced by 90 µl of 2% FBS-containing Humedia EB2 (Kurabo), to which was then added 10 µl of additive, and after a lapse of 48 hrs the number of cells was counted. No medium was exchanged during this period. In counting, a tetrazolium salt WST-8 was added to each well (0.5mM) , and after 2 hrs the absorbance was measured at 450nm. The tetrazolium salt added was reduced with an intracellular dehydrogenase to yield formazan depending on the number of cells in the well. In advance, the absorbance at 450nm was confirmed to show a linearity within the range of 500 to 12000 cells in the well.

### «Experiment of luminal formation»

Using a concurrent culture system of human endothelial cells and fibroblasts planted on a 24-well plate (Kurabo Co., Osaka, Japan) , the culture medium was replaced by culture media containing a variety of additives at the early stage of luminal formation. Every 3 days, the medium was exchanged with the additive; 10 day after incubation, the endothelial cell only was stained by using mouse anti-human CD31 antibody (Kurabo Co., Osaka, Japan), goat anti-mouse IgG AlkP Conjugate (Kurabo Co., Osaka, Japan), and BCPI (5-bromo-4-chloro-3-indolyl phosphate) (Kurabo Co., Osaka, Japan) to photograph. The visualized area of the lumen was measured by means of NIH image software.

### «Results of the growth experiment»

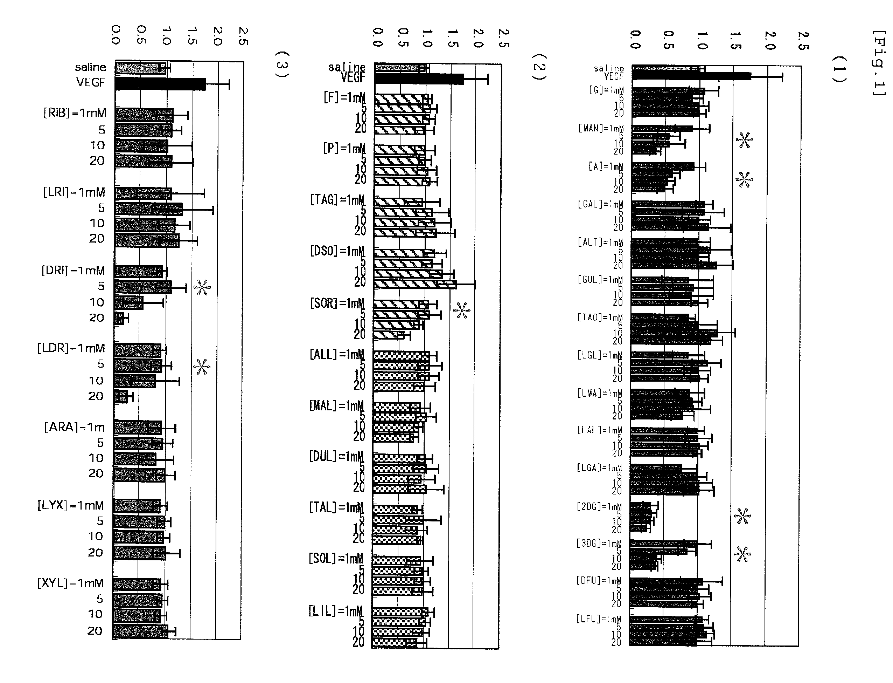

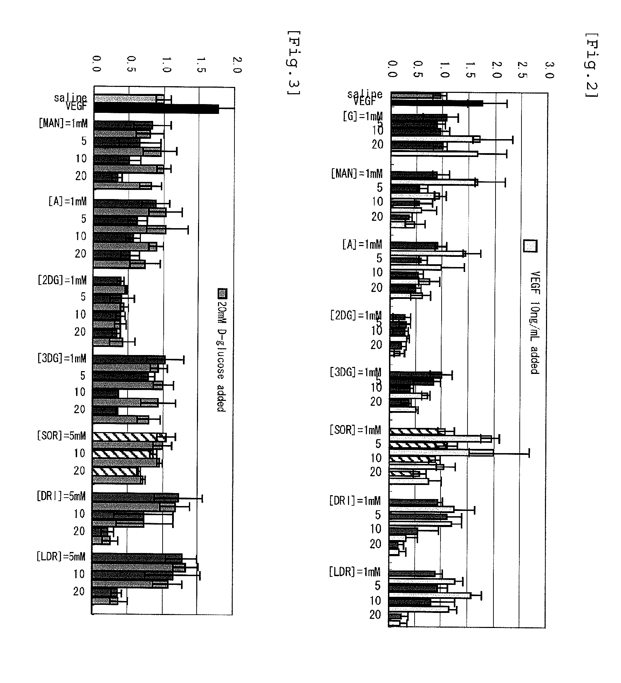

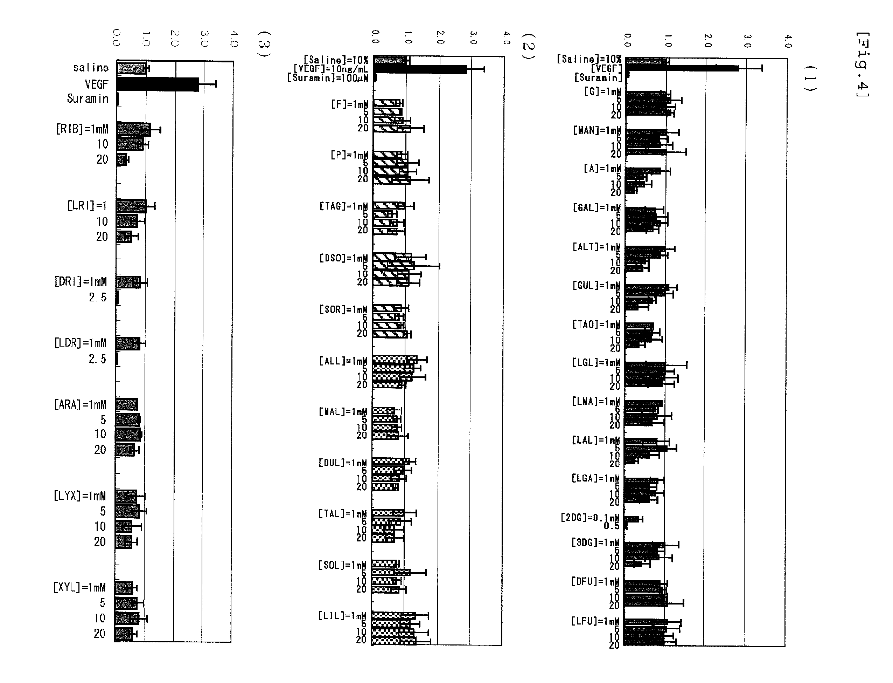

In the control group, 0, 24 and 48 hours after exchange of the medium, the number of the cells was not changed or slightly increased. In the group in which the vascular endothelial cell growth factor VEGF was added (10ng/mL), the cell number was increased 1.7±0.5 times in comparison with the control group. There was no difference between the D-glucose-added groups (1, 5, 10 and 20mM) and the control group.
Among 33 species of sugars tested, an inhibitory effect depending on the concentration was recognized in 7 species, and no effect was observed in 26 species (Fig.1) . The sugars in which the effect was confirmed are MAN, A, 2DG, 3DG, SOR, DRI and LDR. Among them, 6 species except 2DG exhibit no large difference in the potency and depends on the concentration; the inhibition at 20mM was 40-80% to the control group. Only 2DG showed a strong inhibitory effect of 70% at 1mM. In the co-existence of VEGF, these sugars showed a tendency to increase the cell number in comparison to the case of no addition of VEGF, and the inhibitory effect was dependent on the concentration of the sugar compared to the independent effect of VEGF (Fig.2).
In order to consider the mechanism of inhibition, it was examined whether this effect is antagonized by D-glucose. In the co-existence of 20mM D-glucose, the effect of MAN, A and 3DG was antagonized.
However, no antagonism was observed in 2DG, SOR, DRI, and LDR (Fig.3).

### «Result of the experiment of luminal formation»

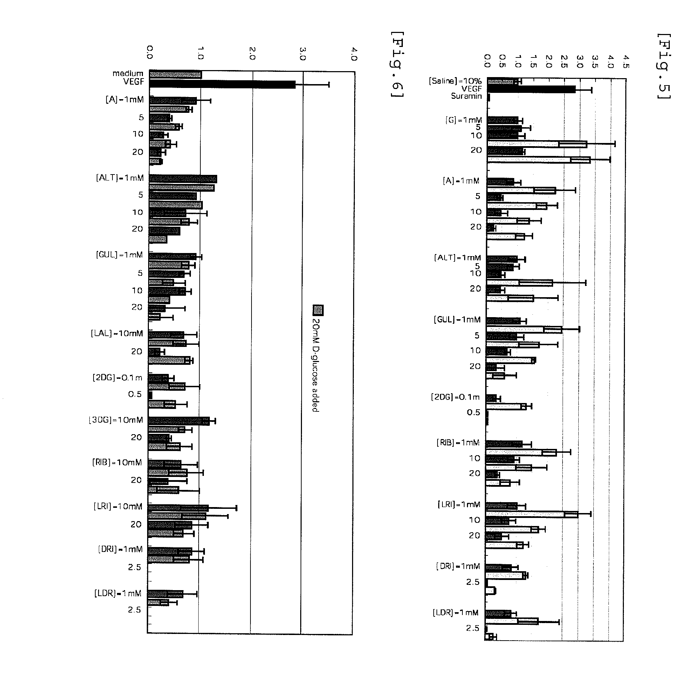

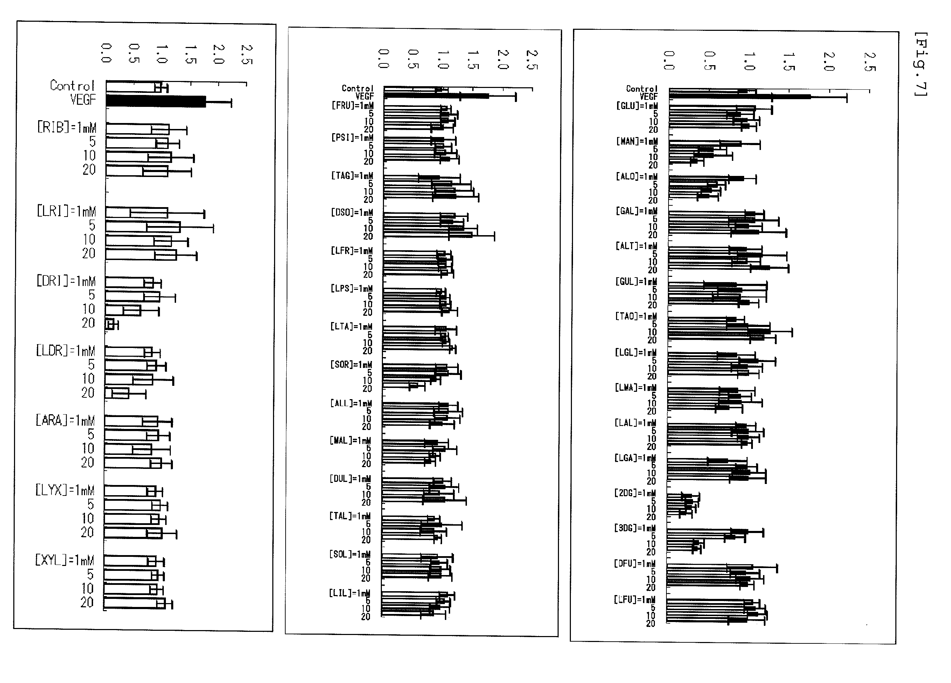

In the group in which 10ng/mL of VEGF was added, the luminal area was enlarged 2.8±0.6 times in comparison to the control group. Among 33 species of sugars tested, an inhibitory effect was recognized in 11 species, and no effect was observed in 22 species (Fig.4). The sugars inwhichtheeffectwasconfirmed are A, ALT, GUL, TAO, LAL, 2DG, 3DG, RIB, LRI, DRI, and LDR. MAN and SOR which showed an inhibitory effect for growth had no influence on the luminal formation. ALT, GUL, TAO, LAL, RIB, and LRI had no influence on the growth, but they inhibited the luminal formation. All of these sugars exhibited a concentration-depending inhibitory effect. A, ALT, GUL, 2DG, RIB, LRI, DRI, and LDR were confirmed to inhibit the acceleration by VEGF, too (Fig.5).
As for the inhibition intensity, A, ALT, GUL, TAO, LAL, 3DG, RIB, and LRI had the same level of intensity, and showed 50-80% inhibition at 20mM. DRI and LDR showed about 20% inhibition at 1mM, but at 2.5mM a strong inhibition over 95%. 2DG had a much stronger inhibitory effect and showed 70% inhibition at 0.1mM, and 95% at 0.5mM. In the co-existence of 20mM D-glucose, the effects of A, ALT, GUL, LAL, 2DG, 3DG, RIB, LRI, DRI, and LDR were examined. Among these sugars, the inhibitory effects of LAL and 2DG were antagonized, and the others had no apparent effect (Fig.6).

### Example 2

To the sugars used in Example 1 was additionally added L-fructose (LFR), L-psicose (LPS), and L-tagatose (LTA), and the same experiment was carried out. Experiment was carried out in the same way as described in Example 1.
The species and abbreviation of the sugars examined are as follows.

### Aldohexose:

D-glucose (GLU); D-mannose (MAN); D-allose (ALO), D-galactose (GAL); D-altrose (ALT); D-gulose (GUL); D-talose (TAO); L-glucose (LGL); L-mannose (LMA); L-allose (LAL); L-galactose (LGA);2-deoxy-D-glucose (2DG) ; 3-deoxy-D-glucose (3DG); 6-deoxy-G-galactose (DFU); 6-deoxy-L-galactose (LFU) = D-fucose

### Ketohexose:

D-fructose (FRU); D-psicose (PSI); D-tagatose (TAG); D-sorbose (DSO) ; L-fructose (LFR) ; L-psicose (LPS) ; L-tagatose (LTA); L-sorbose (SOR)

### Hexitol:

allitol (ALL) ; D-mannitol (MAL) ; dulcitol =galactitol (DUL) ; D-tallitol (TAL); D-sorbitol (SOL); L-iditol (LIL)

### Aldopentose:

D-ribose (RIB) ; D-arabinose (ARA) ; D-lyxose (LYX) ; D-xylose (XYL); L-ribose (LRI); 2-deoxy-D-ribose (DRI); 2-deoxy-L-ribose (LDR)

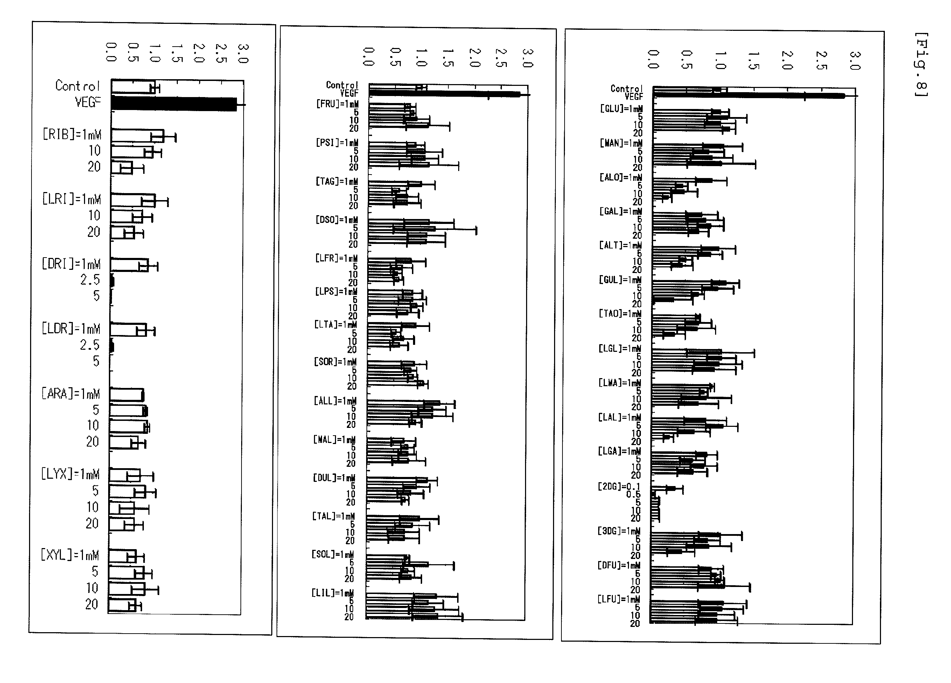

Fig.7 shows the effect of monosaccharides on the growth of HUVEC (48 hours); and Fig.8 shows the effect of monosaccharides on the luminal formation of HUVEC (incubation for 10 days). Among them, the additional data for Example 1 is the bars of ketoses in the middle part of the graph. Among 8 species of ketohexoses, i.e., FRU: D-fructose, PSI: D-psicose, TAG: D-tagatose, DSO: D-sorbose, LFR: L-fructose; LPS: L-psicose, LTA: L-tagatose, and SOR: L-sorbose, SOR showed growth inhibition, and the others had no remarkable effect.
The following 7 species, i.e., MAN, ALO, 2DG, 3DG, SOR, DRI, and LDR had an influence on the growth, in all of which the inhibitory effect was observed. 2DG showed a particularly strong action.
The following 11 species, i.e., ALO, ALT, GUL, TAO, LAL, 2DG, 3DG, RIB, LRI, DRI, and LDR had an influence on the luminal formation. In all of which the inhibitory effect was observed, DRI and LDR had a strong action, and 2DG showed much stronger action.

### Example 3

### «Experiment of glucose up-take in vascular endothelial cell»

Most of the physiological actions of monosaccharides are likely to be caused by the structural similarity to D-glucose, and one of the mechanisms is considered to be an influence on glucose metabolism. Then, in order to elucidate the influence on metabolism, the effect of HUVEC on the glucose up-take was examined for FRU, ALO, PSI, ALL, 2DG, MAN, and GAL. ¹⁴C-Glucose was added to a culture medium, and the incorporated amount after 48 hours was counted by a scintillation counter.

### «Experimental Method»

HUVEC was inoculated on a 24-well plate at a rate of 12000 cells/well. The medium was allowed to stand in 400 *µ*L of Humedia EB2 + 2%FBS overnight. Next day, the medium was replaced by a medium containing ¹⁴C-glucose. The medium used in replacement was the total 400*µ* L/well of medium containing ¹⁴C-glucose (360*µ*L) + saline/VEGF (20*µ*L) + saline/RS (20*µ*L).
The stock solution of ¹⁴C-glucose 100Bq/ *µ*L saline was diluted 10-fold with 2% FBS-containing medium (glucose-free or low glucose) to give a culture medium of 10Bq/ *µ*L.
The experiment was carried out on a lower glucose medium (final concentration [GLU] = 0.1mM) and on a high glucose medium ([GLU] = 4.5mM). ¹⁴C-glucose was added at 3600Bq/well.
Further, FRU: D-fructose, ALO: D-allose, PSI: D-psicose, ALL: allitol, 2DG: 2-deoxy glucose, MAN: D-mannose, or GAL: D-galactose was added to the medium as an additive at 20mM (provided 2mM for 2DC) . Irritation for growth was also carried out with VEGF (10ng/mL).
After a lapse of 48 hours, the well was washed twice with PBS, to which was then added 200µL of 2% Triton, of which 180µL was used as a sample for scintillation counter.

### «Results of experiment»

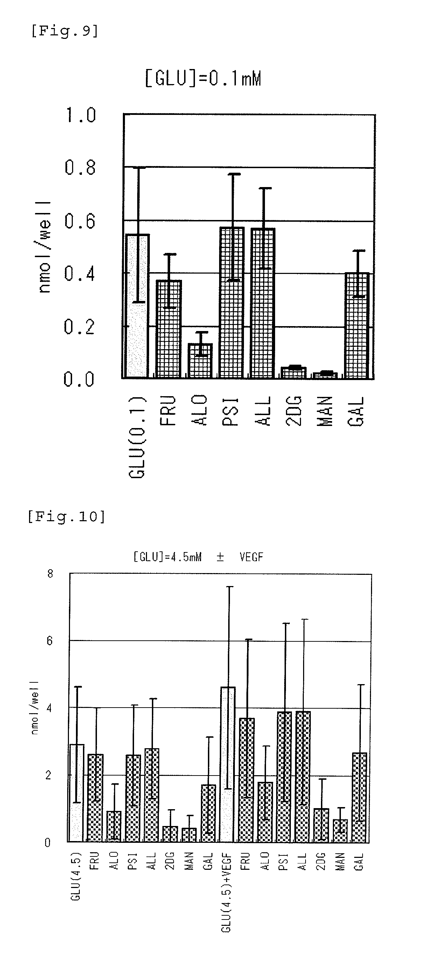

The incorporation of ¹⁴C-glucose into the HUVEC cells was calculated from the count in each well in the unit of nmol/well and depicted in a graph. Fig.9 shows the results of the low glucose medium; and Fig. 10 shows the results of the high glucose medium.
Consequently, the inhibitory effect for the glucose up-take by ALO, 2DG and MAN was observed in both culture media, and no effect was observed in other sugars. VEGF accelerated the glucose up-take and similarly it was inhibited by ALO, 2DG and MAN. These results were identical with the effect on the growth as mentioned above, and thus the inhibition for the growth was considered to be caused by inhibition of the up-take of D-glucose as an energy source.

### «Discussion»

Pentose and hexose take a chain form or a cyclic structure as pyranose or furanose in a solution; the cyclic structure includes □ and □-anomers, each of which can take diverse structures such as chair form, boat form, plain form, envelope form, or their intermediate form. In this situation, it is appropriate to consider a chair form pyranose structure which exists most abundantly. Fig. 11 shows the 4 types of structure of D-glucose.
In case of D-glucose, the chair type C1 occupies almost 100% in an aqueous solution. Though D-glucose does not inhibit the growth inhibition and luminal formation, 2-deoxy-D-glucose and 3-deoxy-D-glucose inhibit both. The only difference in structure between these derivatives and D-glucose resides in that the equatorial hydroxyl group at C2 or C3 of D-glucose is replaced by a hydrogen atom. Accordingly, "(1) an equatorial hydrogen atom at C2 or C3" is considered to be an essential requirementinshowing aninhibitory activity. Among 33 species of the sugars examined, those having "an equatorial hydrogen atom at C2" in the pyranose structure which has been reported to be stable are D-mannose, 2-deoxy-D-glucose, 2-deoxy-D-ribose, 2-deoxy-L-ribose, D-altrose, D-talose, D-ribose,L-ribose,L-mannose,and D-lyxose. The sugars having "an equatorial hydrogen atom at C3" are D-allose, 3-deoxy-D-glucose, D-altrose, D-gulose, L-allose, D-ribose, and L-ribose. These sugars include all of the 13 species in which the inhibitory activity was observed except L-sorbose.
L-Sorbose is the only ketose among the sugars in which the inhibitory activity was observed. Themoststablestructure in 4 species of ketohexoses is regarded as 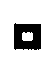 -1C structure for fructose and psicose and 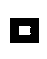 -C1 structure for tagatose and sorbose. and the 1,3-diaxial interaction induced in sorbose is least. In general, when a ketohexose takes pyranose structure, the 1,3-diaxial interaction is readily induced since both of a hydroxyl group and a hydroxymethyl group exist on the carbon atom at 1 position. In case of aldose, it is likely to be more difficult to cause 1,3-diaxial interaction than ketose since one of both is a hydrogen atom. In considering that all of the sugars having an inhibitory activity were aldoses except sorbose and that in sorbose the 1,3-diaxial interaction is least among ketoses, "(2) the least 1,3-diaxial interaction" is considered to be an essential requirement for showing the activity.
In addition, as shown in Table, the sugars having an inhibitory activity take furanose or aldehyde structure in a relatively high rate. Thus, another requirement might be "(3) existence of furanose or aldehyde structure" (Table 1).
Hexitol is not be able to satisfyanyof these 3 requirement. In fact, 6 species of the hexitols examined had no inhibitory activity.
Regarding the first requirement, the phrase "the equatorial substituent attached on some carbon in a monosaccharide is a hydrogen" has the same significance as "the axial substituent attached to said carbon is a hydroxyl group" , and thus the axial hydroxyl group causes 1, 3-diaxial interaction to be against the second requirement. 2-Deoxy-sugars or 3-deoxy-sugars does not produce such an axial hydroxyl group. Accordingly, it is considered that these deoxy-sugars would occupy the 4 species among 5 species of the sugars which inhibited both of the growth and luminal formation.
Though in some cases there are sugars for which the above three requirements could not be applied for explanation, most of sugars could be distinguished according to the requirements. This is considered to be an evidence of the requirements involved in expression of the activity.

| [Table 1] | | | | | | | |
|---|---|---|---|---|---|---|---|
| | Ald type(%) | Pira 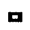 (%) | C1/1C | Pira 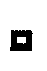 (%) | C1/1C | Fra □ (%) | Fra □ (%) |
| Glucose | 0.024 | 36,36 | C1 | 64 | C1 | | |
| Galactose | 0.082 | 36,27 | C1 | 64 | C1 | trace | trace |
| Allose | 1.38 | 18,20 | C1 | 70 | C1 | 5 | 7 |
| Idose (no exam) | | | 31,46 | mix | 37 | C1 | 16 |
| Fructose | | 28 | mix | 72 | 1C | | |
| Psicose | | 33 | C1 | 67 | 1C | | |
| Tagatose | | 89 | C1 | 11 | C1 | | |
| Xylose | 0.17 | 37,33 | C1 | 63 | C1 | | |
| Arabinose | 0.28 | 63,63 | 1C | 34 | mix | total 3 | |
| Lyxose | 0.4 | 72,71 | mix | 28 | C1 | | |
| Ribose | 8.5 | 20,26 | mix | 56 | mix | 6 | 18 |

| | | | | | | | |
|---|---|---|---|---|---|---|---|
| (dark hatching:inhibition of growth;thin hatching:inhibition of luminal formation; reversal: both inhibited) | | | | | | | |

Though there are some exceptions, it was confirmed that the monosaccharides suppressing the growth of vascular endothelial cell and the luminal formation have the following common structure:
1) Having a hydrogen on the equatorial position with respect to the carbon at 2 or 3 position;
2) Having the structure of less 1,3-diaxial interaction. This is likely to be a basic requirement for anti-tumor agents or wound-curing agents.

### Industrial Applicability

Some of rare sugars have the effects as luminal formation inhibitors since they inhibit the formation of lumen and further as vasculogenesis inhibitors and are useful as approved drugs/quasi drugs, accordingly. Particularly, they are considered to be useful as therapeutic agents for diseases relating vasculogenesis such as cancer, chronic rheumatoid arthritis, diabetic retinopathy, and the like.

Some of rare sugars had a specific inhibitory effect for the growth of vascular endothelial cell and an inhibitory effect for the luminal formation of vascular endothelial cell.
Thus, the rare sugars which are medicaments in the invention are expected to be utilized as therapeutic agents for diseases accompanied by vasculogenesis, for example, diabetic retinopathy, or visual decrease or blindness due to age-related macular degeneration, the growth of solid tumor, pannus formation in chronic rheumatoid arthritis, particularly in cavitas articularis, the growth of synovial membrane in arthrosis, or psoriasis. In addition to the utilization as approved drugs/quasi drugs, the rare sugars may be utilized inaformofsweeteners, flavors, foodadditives, foodmaterials, foods and beverages, foods and beverages for health, and feeds.

### Brief Description of Drawings

Fig. 1 shows the effect of 33 species of monosaccharides on the growth of HUVEC. (1) Aldohexose (15 species); (2) Ketohexose (5 species) and hexitol (6 species) ; (3) Aldopentose (7 species).
Fig.2 shows an interaction between monosaccharides having the growth inhibitory action and VEGF.
Fig.3 shows an interaction between monosaccharides having the growth inhibitory action and 20mM D-glucose.
Fig.4 shows the effect of 33 species of monosaccharides on the formation of lumen. (1) Aldohexose (15 species); (2) Hetohexose (5 species) and hexitol (6 species); (3) Aldopentose (7 species).
Fig. 5 shows an interaction between monosaccharides having the inhibitory action for the formation of lumen and VEGF.
Fig. 6 shows an interaction between monosaccharides having the inhibitory action for the formation of lumen and 20mM D-glucose.
Fig.7 shows 4 types of structure of D-glucopyranose in chair form.
Fig. 8 shows an influence of 36 species of monosaccharides on the growth of HUVEC (48 hours).
Fig. 9 shows an influence of 36 species of monosaccharides on the formation of lumen of HUVEC (culture for 10 days).
Fig.10 shows the results of examination on the effect of the vascular endothelial cell (HUVEC) on the glucose up-take for FRU, ALO, PSI, ALL, 2DG, MAN, and GAL (final concentration [GLU] = 0.1mM).
Fig. 11 shows the results of examination on the effect of the vascular endothelial cell (HUVEC) on the glucose up-take for FRU, ALO, PSI, ALL, 2DG, MAN, and GAL (final concentration [GLU] = 4.5mM).
Fig.12 shows an Izumoring coordinated picture.
Fig. 13 is an illustration for Izumoring C6 in the lower part of Fig.12.

## Claims

1. A method for suppressing the growth of vascular endothelial cell which comprises utilizing an inhibitory effect of D-mannose,D-allose,2-deoxy-D-glucose,3-deoxy-D-glucose, L-sorbose, 2-deoxy-D-ribose and/or 2-deoxy-L-ribose for the growth of vascular endothelial cell.

2. A method for suppressing the growth of vascular endothelial cell as claimed in Claim 1, which comprises utilizing the effect of D-mannose, D-allose, 2-deoxy-D-glucose, 3-deoxy-D-glucose, L-sorbose, 2-deoxy-D-ribose and/or 2-deoxy-L-ribose and/or a derivative thereof and/or a mixture thereof in the form of composition thereof.

3. A method for suppressing the growth of vascular endothelial cell as claimed in Claim 2, wherein the above-mentioned composition is in the form selected from the group consisting of sweeteners, flavors, food additives, food materials, foods and beverages, foods and beverages for health, approved drugs/quasi drugs and feeds containing as active ingredient D-mannose, D-allose, 2-deoxy-D-glucose, 3-deoxy-D-glucose, L-sorbose, 2-deoxy-D-ribose and/or 2-deoxy-L-ribose and/or a derivative thereof and/or a mixture thereof.

4. A method for suppressing the growth of vascular endothelial cell as claimed in Claim 3, wherein the above-mentioned foods and beverages for health are those used for a patient of a disease accompanied by pathological vasculogenesis.

5. A composition having a suppressive effect for the growth of vascular endothelial cell, which comprises as active ingredient D-mannose, D-allose, 2-deoxy-D-glucose, 3-deoxy-D-glucose, L-sorbose, 2-deoxy-D-ribose and/or 2-deoxy-L-ribose and/or a derivative thereof and/or a mixture thereof.

6. A composition as claimed in Claim 5, wherein the above-mentioned composition is in the form selected from the group consisting of sweeteners, flavors, food additives, food materials, foods and beverages, foods and beverages for health, approved drugs/quasi drugs and feeds.

7. A composition as claimed in Claim 6, wherein the above-mentioned foods and beverages for health are those used for a patient of a disease accompanied by pathological vasculogenesis.

8. A method for inhibiting the luminal formation of endothelial cell which comprises utilizing an inhibitory effect of D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose for the luminal formation of vascular endothelial cell.

9. A method for inhibiting the luminal formation of endothelial cell as claimed in Claim 8 , which comprises utilizing the effect of D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose and/or a derivative thereof and/or a mixture thereof in the form of composition thereof.

10. A method for inhibiting the luminal formation of endothelial cell as claimed in Claim 9, wherein the above-mentioned composition is in the form selected from the group consisting of sweeteners, flavors, food additives, food materials, foods and beverages, foods and beverages for health, approved drugs/quasi drugs and feeds containing as active ingredient D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose and/or a derivative thereof and/or a mixture thereof.

11. A method for inhibiting the luminal formation of endothelial cell as claimed in Claim 10, wherein the above-mentioned foods and beverages for health are those used for a patient of a disease accompanied by pathological vasculogenesis.

12. A composition having an inhibitory effect for the luminal formation of vascular endothelial cell, which comprises as active ingredient D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose and/or a derivative thereof and/or a mixture thereof.

13. A composition as claimed in Claim 12, wherein the above-mentioned composition is in the form selected from the group consisting of sweeteners, flavors, food additives, food materials, foods and beverages, foods and beverages for health, approved drugs/quasi drugs and feeds.

14. A composition as claimed in Claim 13, wherein the above-mentioned foods and beverages for health are those used for a patient of a disease accompanied by pathological vasculogenesis.

15. A method for suppressing an abnormal vasculogenesis which comprises utilizing a suppressive effect of D-mannose, D-allose, 2-deoxy-D-glucose, 3-deoxy-D-glucose, L-sorbose, 2-deoxy-D-ribose and/or 2-deoxy-L-ribose for the growth of vascular endothelial cell, and/or an inhibitory effect of D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose for the luminal formation of vascular endothelial cell.

16. A method for suppressing an abnormal vasculogenesis as claimed in Claim 15, which comprises utilizing the effect of D-mannose,D-allose,2-deoxy-D-glucose,3-deoxy-D-glucose, L-sorbose, 2-deoxy-D-ribose and/or 2-deoxy-L-ribose and/or a derivative thereof and/or a mixture thereof in the form of composition thereof, and/or the effect of D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose and/or a derivative thereof and/or a mixture thereof in the form of composition thereof.

17. A method for suppressing an abnormal vasculogenesis as claimed in Claim 16, wherein the above-mentioned composition is in the form selected from the group consisting of sweeteners, flavors, food additives, food materials, foods and beverages, foods and beverages for health, approved drugs/quasi drugs and feeds containing as active ingredient D-mannose, D-allose, 2-deoxy-D-glucose, 3-deoxy-D-glucose, L-sorbose, 2-deoxy-D-ribose and/or 2-deoxy-L-ribose and/or a derivative thereof and/or a mixture thereof, and/or D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose and/or a derivative thereof and/or a mixture thereof.

18. A method for suppressing an abnormal vasculogenesis as claimed in Claim 17, wherein the above-mentioned foods and beverages for health are those used for a patient of diabetes mellitus.

19. A composition for suppressing an abnormal vasculogenesis, which comprises as active ingredient D-mannose, D-allose, 2-deoxy-D-glucose, 3-deoxy-D-glucose, L-sorbose, 2-deoxy-D-ribose and/or 2-deoxy-L-ribose and/or a derivative thereof and/or a mixture thereof, and/or D-allose, D-altrose, D-gulose, D-talose, L-allose, 2-deoxy-D-glucose, 3-deoxy-D-glucose, D-ribose, L-ribose, 2-deoxy-D-ribose and/or 2-deoxy-L-ribose and/or a derivative thereof and/or a mixture thereof.

20. A composition as claimed in Claim 19, wherein the above-mentioned composition is in the form selected from the group consisting of sweeteners, flavors, food additives, food materials, foods and beverages, foods and beverages for health, approved drugs/quasi drugs and feeds.

21. A composition as claimed in Claim 20, wherein the above-mentioned foods and beverages for health are those used for a patient of pathological vasculogenesis.
